# EUROPÄISCHE PATENTSCHRIFT

(11) **EP 0 648 477 B1**
(45) Veröffentlichungstag und Bekanntmachung des Hinweises auf die Patenterteilung: **29.12.1999**
(21) Anmeldenummer: 94113033.8
(22) Anmeldetag: 20.08.1994
(51) Int. Cl.: A61B 17/56, A61B 17/16

(54) **Markraumbohrkopf**
Drill head for the medullary canal
Tête de perçage du canal médullaire

(30) Priorität: 15.09.1993 CH 277493
(43) Veröffentlichungstag der Anmeldung: 19.04.1995
(73) Patentinhaber: Synthes AG, Chur, 7002 Chur (CH)
(72) Erfinder: Müller, Christof, D-13585 Berlin (DE); Christen, Peter, CH-2545 Selzach (CH)
(74) Vertreter: Lusuardi, Werther Giovanni, Dr.

(56) Entgegenhaltungen:
- EP-A- 0 440 371
- EP-A- 0 508 710
- EP-A- 0 532 869
- WO-A-91/09202
- DE-A- 2 838 348

## Beschreibung

Die Erfindung bezieht sich auf einen Markraumbohrkopf gemäss dem Oberbegriff des Patentanspruchs 1.

Bei der osteosynthetischen Nagelung von frakturierten Röhrenknochen muss vor der Implantation des Marknagels die Markhöhle aufgebohrt werden. Zu diesem Zweck wird vorerst ein Bohrdorn durch die Markraumsegmente der einzelnen Knochenfragmente hindurchgeführt, über welchen dann eine hohle, flexible Bohrwelle mit festem Bohrkopf vorgeschoben wird. Der Antrieb der Bohrwelle erfolgt motorisch, zumeist mittels einer pneumatischen Bohrmaschine. Danach wird eine flexible Welle mit austauschbaren Markraumbohrköpfen mit verschieden grossem Aussendurchmesser zum definitiven Aufbohren des Markraumes eingesetzt. Die Markraumbohrköpfe können mittels einer einfachen Kupplung drehfest an der flexiblen Welle befestigt, bzw. von dieser gelöst werden. Ein solcher Markraum bohrkopf ist z.B aus der Druckschrift EP-A-0 440 371 bekannt.

Da die Markraumbohrköpfe nach dem Stand der Technik - abgesehen vom durchlaufenden Kanal für den Bohrdorn, welcher durch diesen selbst verschlossen wird - solide ausgeführt sind, wird beim Aufbohrvorgang ein erheblicher metaphysärer und radialer Druck innerhalb des Markraumes des distalen Frakturfragmentes erzeugt. Dabei können Spitzenwerte für den Druck bis zu 2·10⁵ Pa (1500 mm Hg) auftreten.

Die enorme Druckerhöhung bedingt eine Markfett-Einpressung in die transcorticalen Gefässe, was sich nachteilig auf die Frakturheilung auswirkt, denn die transcorticalen Gefässe werden nicht nur durch den Bohrvorgang selbst, sondern auch noch durch die Verlegung mit Markfett in ihrer Funktion beeinträchtigt. Des weiteren kommt es zu druckbedingten Fetteinschwemmungen in das abführende venöse System, was zu Fettembolien führen kann.

Auch der beim Bohrvorgang auftretende Temperaturanstieg - mit Spitzenwerten bis zu 50°C - ist unerwünscht.

Schliesslich kommt es auch häufig zu einer Verklemmung des Bohrkopfs im Markraum, der dann mit geeigneten Instrumenten wieder herausgeschlagen werden muss.

Hier will die Erfindung Abhilfe schaffen. Der Erfindung liegt die Aufgabe zugrunde, einen Markraumbohrkopf zu schaffen, der die Abführung des hydraulischen Druckes im distalen Frakturfragment während der Aufbohrung des Markraumes gestattet.

Die Erfindung löst die gestellte Aufgabe mit einem Markraumbohrkopf, welcher die Merkmale des Anspruchs 1 aufweist. Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank des erfindungsgemässen Markraumbohrkopfes der beim Bohrvorgang vor dem Bohrkopf auftretende Druck durch die dazu vorgesehenen Öffnungen durch den hohlen Bohrkopf nach hinten abgeführt werden kann.

Die Erfindung wird im folgenden anhand einer Darstellung eines bevorzugten Ausführungsbeispiels noch näher erläutert.

Es zeigt:
Fig. 1 eine perspektivische Darstellung einer bevorzugten Ausführungs form des Markraumbohrkopfes;
Fig. 2 eine schematische Vorderansicht des Markraumbohrkopfes nach Fig. 1 mit Darstellung der Grösse und Winkellage der Öffnungen, die symbolisch angedeutet sind;
Fig. 3 eine schematische Vorderansicht einer Variante des Markraumbohrkopfes nach Fig. 2; und
Fig. 4 eine vereinfachte, schematische Seitenansicht des Markraumbohrkopfes nach Fig. 1 mit Darstellung der Winkellage einer der Öffnungen.

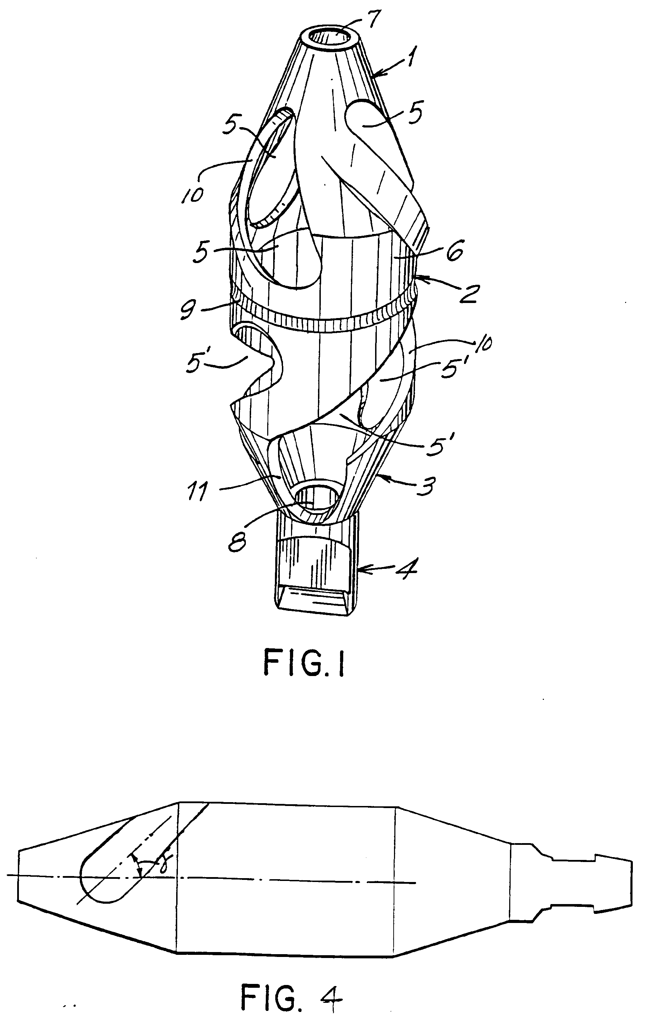

Der in Fig. 1 dargestellte Markraumbohrkopf besteht im wesentlichen aus einem hohlen Rotationskörper mit einem kegelstumpfförmigen Stirnteil 1, einem Mittelteil 2, einem kegelstumpfförmigen Hinterteil 3 und einem am Hinterteil 3 anschliessenden Kupplungsteil 4 zur lösbaren Befestigung an eine (zeichnerisch nicht dargestellte) antreibbare, flexible Bohrwelle. Die Wandstärke des Mantels des hohlen Rotationskörpers beträgt zweckmässigerweise ca. 1 mm.

Der Stirnteil 1 und der Hinterteil 3 (mit Kupplungsteil 4) besitzen je eine Austrittsöffnung 7, bzw. 8, durch welche ein (zeichnerisch nicht dargestellter) Bohrdorn oder eine Bohrerführung mit gleichem Durchmesser (von ca. 3 mm) gesteckt werden kann.

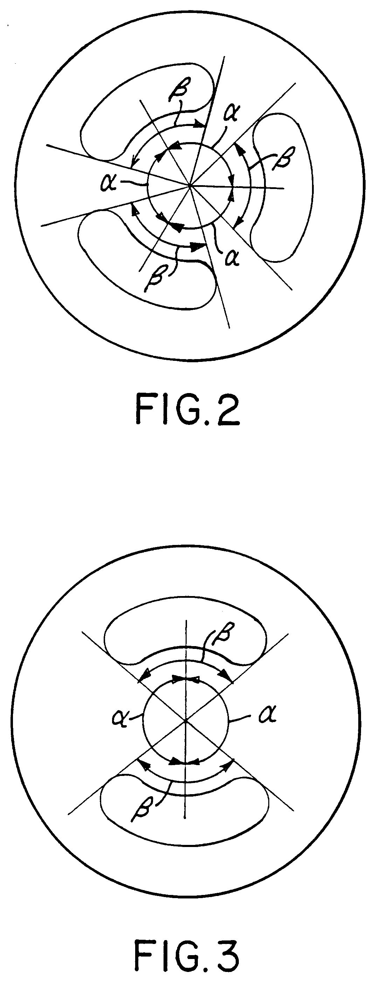

Im Stirnteil 1 sowie im daran angrenzenden Abschnitt des Mittelteils 2 sind in der Mantelfläche 6 des hohlen Rotationskörpers drei Öffnungen 5 in Form von um 120° versetzt angeordneten, spiralförmig verlaufenden Schlitzen angebracht. Mit anderen Worten beträgt der Winkelabstand α - wie in Fig. 2 dargestellt - zwischen zwei identischen Punkten, beispielsweise das Zentrum oder eines der beiden Enden, zweier benachbarter Schlitze 120°.

Annähernd symmetrisch dazu sind im Hinterteil 3 sowie im angrenzenden Abschnitt des Mittelteils 2 ebenfalls drei Öffnungen 5' in Form von um 120° versetzten, spiralförmig verlaufenden Schlitzen angebracht. Wie in Fig. 2 dargestellt erstrecken sich die Öffnungen 5,5' über einen Sektor β von 60° - 120°, vorzugsweise von 80° - 100° und typischerweise von 90°. An ihrem hinteren Ende sind die Schlitze des Hinterteils 3 wie an der Stelle 11 angedeutet etwas erweitert (z.B. in Form einer sphärischen Ausnehmung) um eine Verstopfung des Markraumbohrkopfs zu verhindern.

Wie in Fig. 4 dargestellt liegt der Steigungswinkel gamma, d.h. die Inklination der spiralförmig verlaufenden Schlitze relativ zur Längsachse des Markraumbohrkopfes, im Bereich zwischen 20° und 40°, vorzugsweise zwischen 25° und 35° und beträgt typischerweise 30°.

Anstelle der drei um 120° versetzten Schlitze können - wie in Fig. 3 dargestellt - auch zwei um 180° versetzte Schlitze verwendet werden.

Die Öffnungen 5,5' im Form von Schlitzen sind derart ausgebildet, dass sie schneidende Kanten 10 aufweisen, analog zu einer Raffel, wobei vorzugsweise die zum Kupplungsteil 4 gerichteten Kanten 10 radial erhöht sind, beispielsweise durch Umbiegen derselben.

Der Kupplungsteil 4 ist gemäss dem Stand der Technik ausgebildet und kann mit irgend einem geeigneten Kupplungssystem für die daran zu befestigende Bohrwelle ausgestattet werden.

Fabrikationstechnisch lassen sich die beiden Hälften des hohlen Rotationskörpers getrennt herstellen und dann längs der Schweissnaht 9 miteinander verbinden.

Die Wandstärke des Mantels des hohlen Rotationskörpers kann zwischen 0,5 und 2 mm, vorzugsweise zwischen 0,8 und 1,2 mm betragen.

Der Markraumbohrkopf ist zweckmässigerweise zu mindestens 50%, vorzugsweise mindestens 70% seines Gesamtvolumens ausgehölt.

## Patentansprüche

1. Markraumbohrkopf zum Aufbohren der Markhöhle eines Röhrenknochens mit einem Stirnteil (1), einem Mittelteil (2), einem Hinterteil (3) und einem am Hinterteil anschliessenden Kupplungsteil (4) zur lösbaren Befestigung an eine antreibbare Bohrwelle, dadurch gekennzeichnet, dass der Markraumbohrkopf als hohler Rotationskörper ausgebildet ist und mindestens im Stirnteil (1) und im Hinterteil (3) eine oder mehrere Öffnungen (5) in der Mantelfläche (6) des hohlen Rotationskörper vorgesehen sind.

2. Markraumbohrkopf nach Anspruch 1, dadurch gekennzeichnet, dass der Stirnteil (1) und vorzugsweise auch der Hinterteil (3) als Hohlkegelstumpf ausgebildet ist.

3. Markraumbohrkopf nach Anspruch 1 oder 2, dadurch gekennzeichnet, dass die Öffnungen (5) im Form von spiralförmig in der Mantelfläche (6) angeordneten Schlitzen realisiert sind, deren zum Kupplungsteil (4) gerichteten Kanten (10) vorzugsweise schneidend ausgebildet sind.

4. Markraumbohrkopf nach Anspruch 3, dadurch gekennzeichnet, dass im Stirnteil (1) sowie im daran angrenzenden Abschnitt des Mittelteils (2) zwei um 180° gegeneinander versetzte Schlitze und im Hinterteil (3) sowie im angrenzenden Abschnitt des Mittelteils (2) ebenfalls zwei um 180° versetzte Schlitze angeordnet sind.

5. Markraumbohrkopf nach Anspruch 3, dadurch gekennzeichnet, dass im Stirnteil (1) sowie im daran angrenzenden Abschnitt des Mittelteils (2) drei um 120° versetzte Schlitze und im Hinterteil (3) sowie im angrenzenden Abschnitt des Mittelteils (2) ebenfalls drei um 120° versetzte Schlitze angeordnet sind.

6. Markraumbohrkopf nach einem der Ansprüche 3 bis 5, dadurch gekennzeichnet, dass die spiralförmig angeordneten Schlitze einen Steigungswinkel von 20° - 40°, vorzugsweise von 25° - 35° aufweisen.

7. Markraumbohrkopf nach einem der Ansprüche 3 bis 6, dadurch gekennzeichnet, dass die spiralförmig angeordneten Schlitze sich über einen radialen Drehwinkel von 60° - 120°, vorzugsweise von 80° - 100° erstrecken.

8. Markraumbohrkopf nach einem der Ansprüche 1 bis 7, dadurch gekennzeichnet, dass die Stärke des Mantels zwischen 0,5 und 2 mm, vorzugsweise zwischen 0,8 und 1,2 mm liegt.

9. Markraumbohrkopf nach einem der Ansprüche 1 bis 8, dadurch gekennzeichnet, dass der Stirnteil (1) und der Kupplungsteil (4) beide eine Austrittsöffnung (7, 8) aufweisen, durch welche in axialer Richtung ein Bohrdorn führbar ist.

10. Markraumbohrkopf nach einem der Ansprüche 1 bis 9, dadurch gekennzeichnet, dass mindestens 50%, vorzugsweise mindestens 70% seines Gesamtvolumens ausgehöhlt ist.

## Claims

1. Drill head for drilling out the medulla of a tubular bone with a front part (1), a middle part (2), a rear part (3) and connected to the rear part coupling means (4) for a loosenable attachment to a drill shaft which is apt to be driven,
characterized in that
the drill head is formed as a hollow body of revolution and in that it is provided at least at the front part (1) and at the rear part (3) with one or more openings (5) in the lateral area (6) of the hollow body of revolution.

2. Drill head according to claim 1, characterized in that the front part (1) and preferably the rear part (3) as well have the shape of a hollow truncated cone.

3. Drill head according to claim 1 or 2, cnaracterized in that the openings (5) are realized in the form of slots arranged spirally in the lateral area (6) and whose edges (10) facing the coupling means (4) are preferably shaped as cutting edges.

4. Drill head according to claim 3, characterized in that in the front part (1) as well as in the adjacent section of the middle part (2) two slots about 180° apart and also in the rear part (3) as well as in the adjacent section of the middle part (2) two slots about 180° apart are arranged.

5. Drill head according to claim 3, characterized in that in the front part (1) as well as in the adjacent section of the middle part (2) three slots about 120° apart and also in the rear part (3) as well as in the adjacent section of the middle part (2) three slots about 120° apart are arranged.

6. Drill head according to one of the claims 3 to 5, characterized in that the spiral slots are provided with an angle of inclination of 20° to 40°, preferably of 25° to 35°.

7. Drill head according to one of the claims 3 to 6, characterized in that the spiral slots extend over a radial azimuth angle of 60° to 120°, preferably of 80° to 100°.

8. Drill head according to one of the claims 1 to 7, characterized in that the thickness of the shell amounts between 0,5 and 2 mm, preferably between 0,8 and 1,2 mm.

9. Drill head according to one of the claims 1 to 8, characterized in that the front part (1) and the coupling means (4) are both provided with an outlet opening (7;8) where through a drilling thom may be guided in the axial direction.

10. Drill head according to one of the claims 1 to 9, characterized in that at least 50%, preferably at least 70% of its total volume is hollow.

## Revendications

1. Tête de forage de l'espace médullaire, pour le forage du creux médullaire d'un os tubulaire, laquelle tête de forage comporte une partie frontale (1), une partie médiane (2), une partie arrière (3) et une pièce d'accouplement (4) qui se raccorde à la partie arrière pour permettre sa fixation libérable à un arbre de forage apte à être entraîné, caractérisée en ce que la tête de forage de l'espace médullaire est configurée comme corps rotatif creux et en ce qu'au moins dans la partie frontale (1) et dans la partie arrière (3), une ou plusieurs ouvertures (5) sont prévues dans la surface d'enveloppe (6) du corps rotatif creux.

2. Tête de forage de l'espace médullaire selon la revendication 1, caractérisée en ce que la partie frontale (1) et de préférence également la partie arrière (3) sont configurées en tronc de cône creux.

3. Tête de forage de l'espace médullaire selon la revendication 1 ou 2, caractérisée en ce que les ouvertures (5) sont réalisées sous la forme de fentes disposées en spirales dans la surface d'enveloppe (6) et dont les arêtes (10) dirigées vers la pièce d'accouplement (4) sont de préférence configurées de manière à être tranchantes.

4. Tête de forage de l'espace médullaire selon la revendication 3, caractérisée en ce que deux fentes décalées l'une par rapport à l'autre de 180° sont disposées dans la partie frontale (1) ainsi que dans le tronçon de la partie médiane (2) qui s'y raccorde, et en ce que deux fentes décalées de 180° sont également disposées dans la partie arrière (3) ainsi que dans le tronçon de la partie médiane (2) qui s'y raccorde.

5. Tête de forage de l'espace médullaire selon la revendication 3, caractérisée en ce que trois fentes décalées de 120° sont disposées dans la partie frontale (1) ainsi que dans le tronçon de la partie médiane (2) qui s'y raccorde, et en ce que trois fentes décalées de 120° sont également disposées dans la partie arrière (3) ainsi que dans le tronçon de la partie médiane (2) qui s'y raccorde.

6. Tête de forage de l'espace médullaire selon l'une des revendications 3 à 5, caractérisée en ce que les fentes disposées en spirales présentent un angle de pas de 20° à 40°, de préférence de 25° à 35°.

7. Tête de forage de l'espace médullaire selon l'une des revendications 3 à 6, caractérisée en ce que les fentes disposées en spirales s'étendent sur un angle de rotation du rayon de 60° à 120°, de préférence de 80° à 100°.

8. Tête de forage de l'espace médullaire selon l'une des revendications 1 à 7, caractérisée en ce que l'épaisseur de l'enveloppe est comprise entre 0,5 et 2 mm, de préférence entre 0,8 et 1,2 mm.

9. Tête de forage de l'espace médullaire selon l'une des revendications 1 à 8, caractérisée en ce que la partie frontale (1) et la pièce d'accouplement (4) présentent toutes deux une ouverture de sortie (7, 8) par lesquelles une tige de forage peut être guidée dans la direction axiale.

10. Tête de forage de l'espace médullaire selon l'une des revendications 1 à 9, caractérisée en ce qu'au moins 50 %, de préférence au moins 70 % de son volume total sont évidés.
